# EUROPEAN PATENT APPLICATION

(11) **EP 2 065 057 A2**
(43) Date of publication of application: **03.06.2009**
(21) Application number: 09003422.4
(22) Date of filing: 14.03.2007
(51) Int. Cl.: A61K 49/00, A61K 51/04, A61K 51/08, A61K 47/48

(54) **Chelating conjugates having a substituted aromatic moiety and derivatives thereof**

(30) Priority: 15.03.2006 US 782366 P
(62) Divisional of application: 07753161.4
(71) Applicant: Mallinckrodt Inc., Hazelwood, MO 63042 (US)
(72) Inventor: Moore, Dennis, A., St. Louis Missouri 63135 (US)
(74) Representative: Jones, Nicholas Andrew

(57) **Abstract**

A conjugate comprising a bio-directing carrier, a metal coordinating moiety, and a linker chemically linking the metal coordinating moiety to the carrier, the metal coordinating moiety comprising at least one optionally substituted 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl, 2-thiol-4-hydroxypyridyl, 1,3-dithiolphenyl, 1-hydroxy-3-thiolphenyl or 1-thiol-3-hydroxyphenyl moiety, or a combination thereof.

## Description

### BACKGROUND

The present invention is generally directed to metal chelating conjugates for use as a metallopharmaceutical diagnostic or therapeutic agent.

Metallopharmaceutical diagnostic and therapeutic agents are finding ever-increasing application in biological and medical research, and in diagnostic and therapeutic procedures. Generally, these agents contain a radioisotope or paramagnetic metal which upon introduction to a subject, become localized in a specific organ, tissue or skeletal structure of choice. When the purpose of the procedure is diagnostic, images depicting the *in vivo* distribution of the radioisotope, paramagnetic or radioopaque metal can be made by various means, including single photon emission, magnetic resonance and x-ray, depending on the metal selected and substitution pattern on the metal complex. The distribution and corresponding relative intensity of the detected radioisotope, paramagnetic or radioopaque metal not only indicates the space occupied by the targeted tissue, but may also indicate a presence of receptors, antigens, aberrations, pathological conditions, and the like. When the purpose of the procedure is therapeutic, the agent typically contains a radioisotope and the radioactive agent delivers a dose of radiation to the local site.

Depending upon the target organ or tissue of interest and the desired diagnostic or therapeutic procedure, a range of metallopharmaceutical agents may be used. One common form is a conjugate comprising a radioactive or paramagnetic metal, a carrier agent for targeting the conjugate to a specific organ or tissue site, and a linkage for chemically linking the metal to the carrier. In such conjugates, the metal is typically associated with the conjugate in the form of a coordination complex, more typically as a chelate of a macrocycle. See, *e.g.*, Liu, U.S. Patent No. 6,916,460.

In U.S. Patent No. 6,143,274, Tweedle et al. disclose a method for imaging mammalian tissue utilizing a non-ionic complex of a paramagnetic ion of a lanthanide element and a macrocyclic chelating agent. A non-ionic complex, however, is less stable than an anionic complex (i.e., the anionic complex tends to exhibit stronger electrostatic interaction between the cationic metal and anionic ligand).

While metallopharmaceuticals utilizing metal coordinating moieties having a hydroxybenzyl group to assist in the coordination are known, see e.g., A. Martell and R. Smith, Critical Stability Constants, vol. 1: Amino Acids, Plenum Press (1974) (describing HBED), the need for creating metal coordinating groups that demonstrate higher affinity for metals remains important to reduce the overall toxicity of these compounds.

### SUMMARY

Among the several aspects of the present invention is the provision of a conjugate for use in diagnostic and therapeutic procedures. Advantageously, such conjugates tend to accumulate in the specific organ, tissue or skeletal structure with a reduced risk of non-specific binding to non-target tissues, thereby allowing for the conjugates to be targeted to specific disease states, if desired. Further, these conjugates may be formed at relatively low temperatures, thereby decreasing the chance that a carrier for targeting the conjugate to a biological tissue or organ will be destroyed during the complexation reaction.

Briefly, therefore, the present invention is directed to a conjugate, the conjugate comprising one or more carriers for targeting the conjugate to a biological tissue or organ, a metal coordinating moiety, and a linker chemically linking the metal coordinating moiety to the carrier. The metal coordinating moiety comprises at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof.

The present invention is further directed to a conjugate, the conjugate comprising one or more carriers for targeting the conjugate to a biological tissue or organ, a metal coordinating moiety, a metal complexed by the metal coordinating moiety, and a linker chemically linking the metal coordinating moiety to the carrier. The metal coordinating moiety comprises at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof.

The present invention is further directed to a diagnostic or therapeutic method. The method comprises administering a conjugate to a subject. The conjugate comprises one or more carriers for targeting the conjugate to a biological tissue or organ, a metal coordinating moiety, a radioactive or paramagnetic metal complexed by the metal coordinating moiety, and a linker chemically linking the metal coordinating moiety to the carrier, the metal coordinating moiety comprising at least one optionally substituted 1 ,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof.

The present invention is further directed to a kit for the preparation of a metallopharmaceutical. The kit comprises a conjugate for use in a diagnostic or therapeutic method. The conjugate comprises one or more carriers for targeting the conjugate to a biological tissue or organ, a metal coordinating moiety, a linker chemically linking the metal coordinating moiety to the carrier, and, optionally, a metal to be complexed by the metal coordinating moiety, the metal coordinating moiety comprising at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof.

Other aspects of the invention will be in part apparent and in part pointed out hereinafter.

### DETAILED DESCRIPTION OF VARIOUS EMBODIMENTS

The present invention provides conjugates that can rapidly form coordination complexes with metals for use in diagnostic or therapeutic metalloradiopharmaceuticals, or magnetic resonance imaging contrast agents. The conjugates can also serve as bifunctional chelators (BFC's) for attaching metal ions to bio-directing carriers, sometimes referred to as biomolecules, that bind *in vivo* to a tissue type, organ or other biologically expressed composition or receptor. The target-specific metallopharmaceuticals of the present invention are useful in the diagnosis of disease by magnetic resonance imaging or scintigraphy or in the treatment of disease by systemic radiotherapy.

Generally, the conjugates of the present invention comprise a bio-directing carrier and a metal coordinating moiety covalently joined indirectly through a linker, the linker being chemically bonded to the metal coordinating moiety. The metal coordinating moiety comprises (i) a metal chelator and (ii) at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof (sometimes collectively referred to as ((di)thio)dihydroxyaromatic). While not required, the linker may be bonded to the metal coordinating moiety via a ((di)thio)dihydroxyaromatic moiety. Thus, for any given conjugate, the metal coordinating moiety comprises one or more ((di)thio)dihydroxyaromatic moieties, the linker being bonded to the metal coordinating moiety optionally through one of the ((di)thio)dihydroxyaromatic moieties. The particular location(s) of the ((di)thio)dihydroxyaromatic moiety(ies) on the metal coordinating moiety is not critical.

The metal coordinating moiety of the present invention compises at least one ((di)thio)dihydroxyaromatic moiety. Typically, the metal coordinating moiety will comprise multiple ((di)thio)dihydroxyaromatic moieties. In one embodiment, the metal chelator will be linked to the bio-directing carrier via a ((di)thio)dihydroxyaromatic moiety. In another embodiment, the metal chelator will be linked to the bio-directing carrier via a conventional means other than through a ((di)thio)dihydroxyaromatic moiety.

The ((di)thio)dihydroxyaromatic moieties of the present invention comprise a six-membered aryl ring wherein at least four of the ring atoms are carbon atoms and the ring is substituted by two hydroxy, two thiol, or one hydroxy and one thiol group. Ring carbon atoms are located (i) at the point of attachment to the metal chelator and (ii) at the two ring positions alpha to the point of attachment to the metal chelator. The three remaining ring atoms, both ring atoms beta to the carbon atom at the point of attachment to the metal chelator and the ring atom gamma to the carbon atom at the point of attachment to the metal chelator, are independently carbon or nitrogen provided, however, that the aromatic portion of a ((di)thio)dihydroxyaromatic moiety is either phenyl, pyridyl, or pyrimidyl. The hydroxy and/or thiol groups are located at the two carbon atoms alpha to the carbon at the point of attachment to the metal chelator. In addition, any substitutable ring carbon atom may be substituted with a group that influences stability and/or biodistribution and optionally is bonded to a bio-directing carrier via a linker. For example, when a ring atom beta to the carbon atom at the point of attachment of the metal chelator is selected to be carbon, it is available for substitution. Thus, when the aromatic moiety is phenyl, there are three substitutable ring carbon atoms available for substitution. Likewise, when the aromatic moiety is pyridyl, there are two substitutable ring carbon atoms available for substitution. When the aromatic moiety is pyrimidyl, one substitutable ring carbon atom is available for substitution.

In one embodiment, the metal coordinating moiety of the present invention has the general Formula (1): 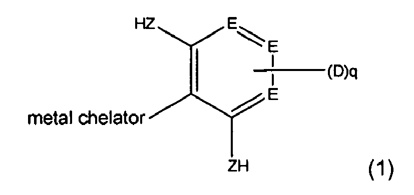 wherein

each E is independently carbon or nitrogen provided, however, that the ring comprising the E atoms is phenyl, pyridyl, or pyrimidyl;

each Z is independently oxygen or sulfur; and

q is 0 to 3 wherein when q is greater than 0, each D is selected to influence stability and/or biodistribution and/or optionally bonds a bio-directing carrier via a linker to the metal chelator.

As previously discussed, the bio-directing carrier may be linked to the metal coordinating moiety via a ((di)thio)dihydroxyaromatic moiety or, alternatively, the bio-directing carrier may be bonded to the metal coordinating moiety via any known conventional means other than through a ((di)thio)dihydroxyaromatic moiety. Thus, schematically, a conjugate comprising the bio-directing carrier, the linker, and the metal coordinating moiety comprising a ((di)thio)dihydroxyaromatic moiety of the present invention may correspond to Formula (A) (where the bio-directing carrier is not linked to the metal chelator via a ((di)thio)dihydroxyaromatic moiety) 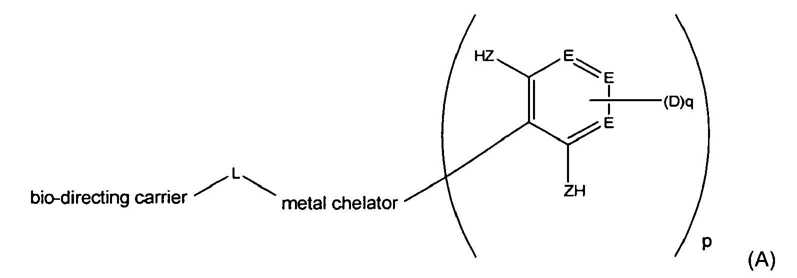 or Formula (B) (where the bio-directing carrier is linked to the metal chelator via a ((di)thio)dihydroxyaromatic moiety) 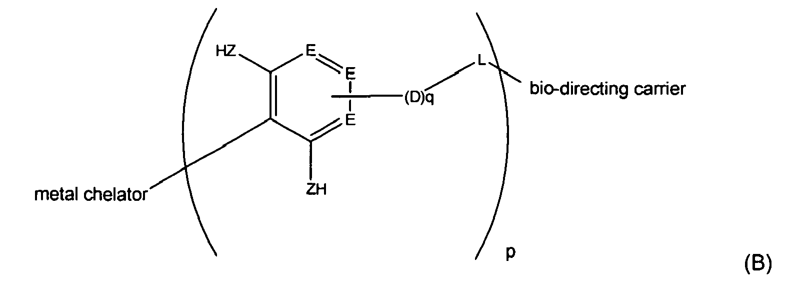 wherein

L covalently bonds, either directly or indirectly, the metal coordinating moiety to the bio-directing carrier;

each Z is independently oxygen or sulfur;

each E is independently carbon or nitrogen provided, however, that the ring comprising the E atoms is phenyl, pyridyl, or pyrimidyl;

q is 0 to 3 wherein when q is greater than 0, each D is selected to influence stability and/or biodistribution and/or optionally bonds a bio-directing carrier via a linker to the remainder to the metal coordinating moiety; and

p is 1 to 5.

Although Formula (A) and Formula (B) depict only a single bio-directing carrier, it is contemplated that a conjugate may comprise multiple bio-directing carriers, each of which is connected to the metal coordinating moiety via the linker, L. Thus, the linker may comprise substituents, each of which is linked to a metal chelating moiety that may be the same or different.

Without being held to any particular theory, it is believed that the orientation of the hydroxyl and/or thiol groups of the ((di)thio)dihydroxyaromatic moiety at the two positions alpha to the carbon at the point of attachment of the metal chelator offers a more robust coordination environment for the metal. For example, it is known that yttrium-oxygen coordination bonds are quite labile. Thus, in solution this bond is breaking and reforming very rapidly. By having a second, positionally equivalent phenolic oxygen available (for a dihydroxyaromatic moiety), however, allows for quick reformation of the bond. Consequently, the second oxygen provides an intramolecular competitive binding event versus any external competition, which could lead to decomplexation and decomposition of the radioisotope. Similarly, because many metals form stable coordination bonds with thiol groups, one or both of the hydroxyl groups may be replaced with thiol groups depending on the nature of the metal selected.

Prior to use in a diagnostic or therapeutic procedure, a conjugate corresponding to Formula (A) or Formula (B) is complexed with a metal to form a metallopharmaceutical diagnostic or therapeutic agent of the present invention.

### Bio-directing Carriers

As previously noted, conjugates of the present invention include one or more bio-directing carriers, also known as biomolecules, that direct the conjugate to the desired tissue, organ, receptor or other biologically expressed composition target. Ideally, the carrier is selective or specific for the targeted organ or tissue site.

Typical bio-directing carriers include hormones, amino acids, peptides, peptidomimetics, proteins, nucleosides, nucleotides, nucleic acids, enzymes, carbohydrates, glycomimetics, lipids, albumins, mono- and polyclonal antibodies, receptors, inclusion compounds such as cyclodextrins, and receptor binding molecules, e.g., αᵥβ₃. Specific examples of carriers include steroid hormones for the treatment of breast and prostate lesions; somatostatin, bombesin, CCK, and neurotensin receptor binding molecules for the treatment of neuroendocrine tumors; CCK receptor binding molecules for the treatment of lung cancer; ST receptor and carcinoembryonic antigen (CEA) binding molecules for the treatment of colorectal cancer; dihyroxyindolecarboxylic acid and other melanin producing biosynthetic intermediates for the treatment of melanoma; integrin receptor and atherosclerotic plaque binding molecules for the treatment of vascular diseases; and amyloid plaque binding molecules for the treatment of brain lesions. Exemplary bio-directing carriers also include synthetic polymers such as polyaminoacids, polyols, polyamines, polyacids, oligonucleotides, aborols, dendrimers, and aptamers.

In one embodiment, the bio-directing carrier is selected from among amides, ethers, antibodies (e.g., NeutroSpect®, Zevalin®, and Herceptin®), proteins (e.g., TCII, HSA, annexin, and Hb), peptides (e.g., octreotide, bombesin, neurotensin, and angiotensin), nitrogen-containing simple or complex carbohydrates (e.g., glucosamine and glucose), nitrogen-containing vitamins (e.g., vitamin A, B1, B2, B12, C, D2, D3, E, H, and K), nitrogen-containing hormones (e.g., estradiol, progesterone, and testosterone), nitrogen-containing active pharmaceuticals (e.g., celecoxib or other nitrogen-containing NSAIDS, AMD3100, CXCR4 and CCR5 antagonists) or nitrogen-containing steroids. In one example of this embodiment, the biomolecules are selected from among imidazole, triazole, a peptide, a nitrogen-substituted simple or complex carbohydrate, a nitrogen-substituted vitamin, and a nitrogen-substituted small molecule. In another example, the biomolecules are imidazole, triazole, the N-terminus of a peptide, a nitrogen-substituted simple or complex carbohydrate or a nitrogen-substituted vitamin.

In another embodiment, the bio-directing carrier is added to a reactive ((di)thio)dihydroxyaromatic moiety. For instance, the linker may be selected from imidazole-carbonyl- or triazole-carbonyl, N-hydroxysuccinimide ester, p-nitrophenyl ester or other commonly used leaving groups (see, e.g., Pearson and Roush, Handbook of Reacgents for Organic Synthesis: Activating Agents and Protecting Groups or Bodansky, Peptide Chemistry: A Practical Textbook) presenting a complex with a substitutionally-reactive moiety. The triazole or imidazole or reactive ester may be displaced by the addition of a bio-directing carrier possessing a nucleophilic moiety forming a new urea or amide bond.

To increase specificity for a particular target tissue, organ receptor or other biologically expressed composition, multiple bio-directing carriers may be utilized. In such instances, the bio-directing carriers may be the same or different. For example, a single conjugate may possess multiple antibodies or antibody fragments, which are directed against a desired antigen or hapten. Typically, the antibodies used in the conjugate are monoclonal antibodies or antibody fragments that are directed against a desired antigen or hapten. Thus, for example, the conjugate may comprise two or more monoclonal antibodies having specificity for a desired epitope thereby increasing concentration of the conjugate at the desired site. Similarly and independently, a conjugate may comprise two or more different bio-directing carriers each of which is targeted to a different site on the same target tissue or organ. By utilizing multiple bio-directing carriers in this manner, the conjugate advantageously concentrates at several areas of the target tissue or organ, potentially increasing the effectiveness of therapeutic treatment. Further, the conjugate may have a ratio of bio-directing carriers designed to concentrate the conjugate at a target tissue or organ that optimally achieves the desired therapeutic and/or diagnostic results.

### Linker

As previously noted, the bio-directing carrier(s) are covalently bonded to the metal coordinating moiety via a linker, L. This linker may be, but is not required to be, bonded to the metal coordinating moiety via a ((di)thio)dihydroxyaromatic moiety. When not bonded to the metal coordinating moiety via a ((di)thio)dihydroxyaromatic moiety, the bio-directing carrier may be linked to the metal coordinating moiety via any conventional means. Typical linking groups include, but are not limited to, amide, carboxamide, urea, thiourea, ester, ether, amine, alkyl, aryl, sulfito, sulfato, phosphito, and phosphate. In one example, the linking group is selected from amide, carboxamide, urea, ether, and alkyl. In another example, the linking group is selected from amide, carboxamide, ether and alkyl.

Further, the linker selected should not interfere with the accumulation of the conjugate in the specific organ, tissue or skeletal structure. In some instances, the linker may actually aid the accumulation of the conjugates of the present invention in the specific organ, tissue or skeletal structure with a reduced risk of non-specific binding to non-target tissues.

The linker may be modified or synthesized such that it bonds to multiple bio-directing carriers and/or affects the biodistribution of the conjugate. For example, the linker may comprise a C₄-C₂₀ carbohydrate moiety, the carbohydrate moiety having the capacity to bind one or more bio-directing carriers through ether linkages. In addition, the carbohydrate moiety increases the water solubility of the conjugate thereby affecting biodistribution.

In one embodiment, L is selected from the group consisting of C₁₋₁₀ alkylene, oxygen, sulfur, keto (-C(O)-), amino (-NH-), amido (-C(O)NH-), urea (-NHC(O)NH-), thiourea (-NHC(S)NH-), ester (-C(O)O-), polyoxo (e.g., -O-CH₂CH₂-O-CH₂CH₂-O-), polyhydroxy (e.g., carbohydrates), and peptides, the alkylene, amino, amido, urea, and thiourea groups being optionally substituted with aryl, C₁₋₇ alkyl, C₁₋₇ hydroxyalkyl or C₁₋₇ alkoxyalkyl. In one example of this embodiment, L is selected from the group consisting of C₁₋₁₀ alkylene, oxygen, sulfur, keto, amino, amido, thiourea, ester, C₄-C₂₀ carbohydrate, the alkylene, amino, amido, and thiourea groups being optionally substituted with aryl, C₁₋₇ alkyl, C₁₋₇ hydroxyalkyl or C₁₋₇ alkoxyalkyl. By way of further example, L may be selected from a more restrictive group, e.g., amido, thiourea, monosaccharides (e.g., hexoses and pentoses) and disaccharides (e.g., sucrose). In one alternative of this embodiment, L comprises other than a urea linkage.

### Metals

Any metal capable of being detected in a diagnostic procedure *in vivo* or *in vitro* or useful in the therapeutic treatment of disease can be employed as a metal in the present conjugates. Particularly, any radioactive metal ion or paramagnetic metal ion capable of producing a diagnostic result or therapeutic response in a human or animal body or in an *in vitro* diagnostic assay may be used. The selection of an appropriate metal based on the intended purpose is known by those skilled in the art. In one embodiment, the metal is selected from the group consisting of Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211. For example, the metal may be selected from the group consisting of Y-90, In-111, Tc-99m, Re-186, Re-188, Cu-64, Ga-67, Ga-68 and Lu-177. By way of further example, the metal may be selected from a more restrictive group, e.g., Y-90, In-111, Tc-99m, Re-186, Cu-64, Ga-67, and Lu-177 or Y-90, In-111, and Tc-99m. In another embodiment, metals that form labile bonds with oxygen, such as yttrium and indium, are appropriate metals for metal coordinating moieties having a ((di)thio)dihydroxyaromatic moiety.

### Metal Coordinating Moiety

The metal coordinating moiety may be any moiety, having at least one ((di)thio)dihydroxyaromatic moiety, used to complex (also referred to as "coordinate") one or more metals under physiological conditions. Preferably, the metal coordinating moiety forms a thermodynamically and kinetically stable complex with the metal to keep the complex intact under physiological conditions; otherwise, systemic release of the coordinated metal may result. Although not required, the oxygen or sulfur atoms comprising the hydroxyl or thiol groups, respectively, of the ((di)thio)dihydroxyaromatic moiety may participate in the complexation of the metal. In other words, the metal coordinating moiety may complex the metal with or without the participation of the hydroxyl or thiol groups of the ((di)thio)dihydroxyaromatic moiety. The participation of the hydroxyl or thiol groups of the ((di)thio)dihydroxyaromatic moiety will depend upon the nature of the metal chelator and the particular metal selected.

In general, the metal coordinating moiety may be acyclic or cyclic. For example, metal coordinating moieties include polycarboxylic acids such as EDTA, DTPA, DCTA, DOTA, TETA, or analogs or homologs thereof. To provide greater stability under physiological conditions, however, macrocyclics, e.g., triaza and tetraza macrocycles, are generally preferred. In some embodiments, the macrocyclic metal coordinating moiety is cyclen or tacn.

In one embodiment, the metal coordinating moiety comprises a substituted heterocyclic ring where the heteroatom is nitrogen. Typically, the heterocyclic ring comprises from about 9 to about 15 atoms, at least 3 of these ring atoms being nitrogen. In one example of this embodiment, the heterocyclic ring comprises 3-5 ring nitrogen atoms where at least one of the ring nitrogen atoms is substituted. For these embodiments, the ring carbon atoms are optionally substituted. One such macrocycle corresponds to Formula (2): 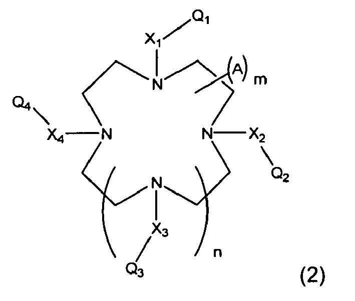 wherein

n is 0, 1 or 2;

m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;

X₁, X₂, X₃ and X₄ are independently optionally substituted methylene where the substituents are selected from the group consisting of aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and

Q₁, Q₂, Q₃ and Q₄ are independently selected from the group consisting of 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D; and

each D is independently selected from the group consisting of a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.

For metal coordinating moieties of Formula (2), the D substituent, if present, is independently bonded to any of the substitutable ring carbon atoms. In one embodiment, each D is fluoro, chloro, bromo, iodo, carboxyl, cyano, nitro, amido, hydroxyl, amino, sulfito, phosphito, sulfato, phosphato, ether, aryl, or C₁₋₈ alkyl optionally substituted with one or more of C₁₋₂₀ alkyl, carboxyl, cyano, nitro, amido, hydroxyl, amino, sulfito, phosphito, sulfato, and phosphate. More typically, each D is bromo, iodo, carboxyl, or hydroxyl. In another embodiment, the metal coordinating moiety is connected to the bio-directing carrier, either directly or indirectly, via a D group.

Typically, for metal coordinating moieties of Formula (2), X₁-X₄ are independently methylene optionally substituted by C₁₋₆ alkyl, halo, or hydroxyl.

In another embodiment of metal coordinating moieties of Formula (2), one of Q₁, Q₂, Q₃ and Q₄ is substituted by D, while the other three of Q₁, Q₂, Q₃ and Q₄ are not substituted by D. Typically, in this embodiment, D is an optionally substituted linker connecting the metal coordinating moiety to one or more bio-directing carriers.

When the metal coordinating moiety corresponds to Formula (2) and m is greater than zero, it is generally preferred that each A be a substituent that positively impacts stability and biodistribution. When present, each A may independently be substituted with one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio substituents. In addition, when A is aryl or alkyl, each of these, in turn, may be optionally substituted with an aryl or C₁₋₂₀ alkyl moiety optionally substituted with one or more aryl, carbaldehyde, keto, carboxyl, cyano, halo, nitro, amido, sulfato, sulfito, phosphato, phosphito, hydroxyl, oxy, mercapto and thio.

Further, for the metal coordinating moieties of Formula (2), the A substituent, if present, is bonded to any of the ring carbon atoms. Further, each ring carbon atom may be substituted by one or two A substituents so that the number of possible A substituents varies with the number of ring carbon atoms. In one embodiment of metal coordinating moieties of Formula (2) having at least one A substituent, each A is independently aryl or C₁₋₈ alkyl optionally substituted with one or more aryl, keto, carboxyl, cyano, nitro, C₁₋₂₀ alkyl, amido, sulfato, sulfito, phosphato, phosphito, oxy and thio. For example, each A may be aryl or C₁₋₆ alkyl optionally substituted with one or more aryl, keto, amido and oxy. By way of further example, each A may be methyl.

In general, as the value of n increases, the size of the macrocycle increases. In this manner, the size of the macrocycle may be controlled to match the size and coordination capacity of the metal to be coordinated.

Exemplary metal coordinating moieties of Formula (2) include: 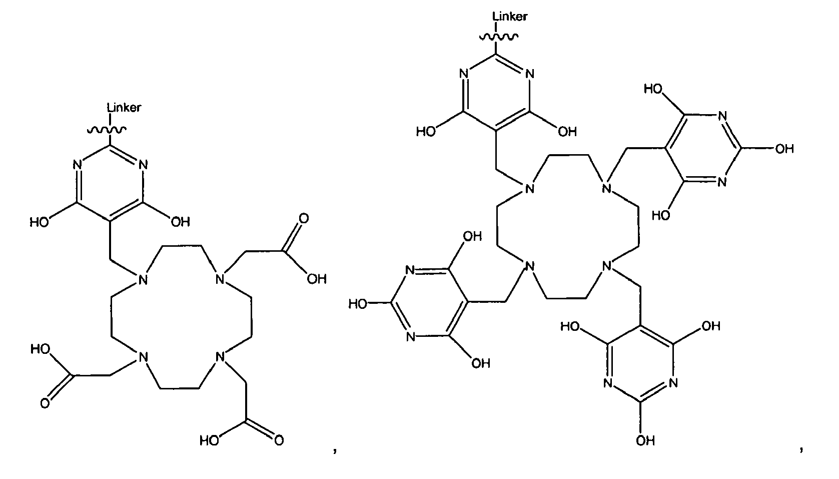 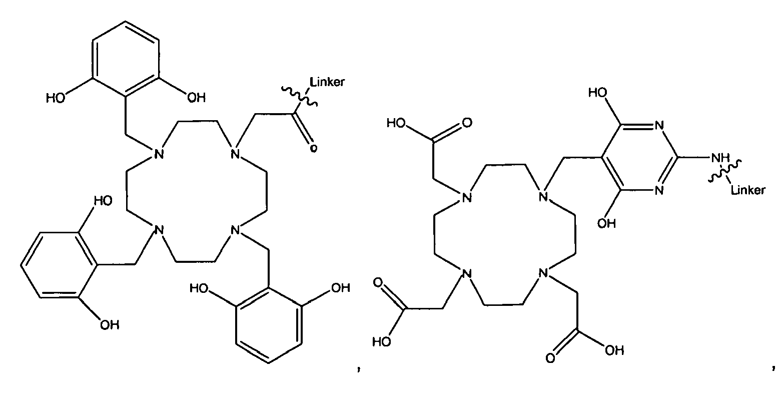 and

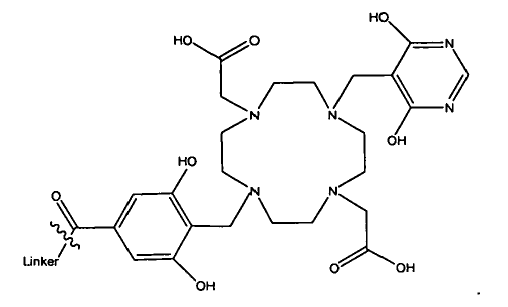

In addition to the metal coordinating moieties comprising a heterocyclic ring, the metal coordinating moieties may alternatively comprise a substituted chain of carbon and nitrogen atoms. As used herein the chain of nitrogen and carbon may be referred to as the "backbone" or the "chain of atoms". Typically, the chain of atoms comprises from about 4 to about 10 atoms, at least 2 of said atoms being nitrogen. Preferably, the chain of atoms comprises 2-4 nitrogen atoms wherein at least one of the chain nitrogen atoms is substituted. The backbone carbon atoms are optionally substituted. Typically, the backbone nitrogen atoms are separated from each other by two carbon atoms. In this embodiment, the metal coordinating moiety typically has the following Formula (3): 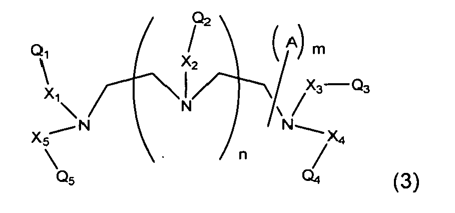 wherein

n is 0, 1 or 2;

m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;

X₁, X₂, X₃, X₄ and X₅ are independently optionally substituted methylene where the substituents are selected from the group consisting of aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and

Q₁, Q₂, Q₃, Q₄, and Q₅ are independently selected from the group consisting of 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄, and Q₅ is 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D; and

each D is independently selected from the group consisting of a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.

For metal coordinating moieties of Formula (3), the D substituent, if present, is independently bonded to any of the substitutable ring carbon atoms. In one embodiment, each D is fluoro, chloro, bromo, iodo, carboxyl, cyano, nitro, amido, hydroxyl, amino, sulfito, phosphito, sulfato, phosphato, ether, C₄-C₂₀ carbohydrate, aryl, or C₁₋₈ alkyl optionally substituted with one or more of C₁₋₂₀ alkyl, carboxyl, cyano, nitro, amido, hydroxyl, amino, sulfito, phosphito, sulfato, and phosphate. More typically, each D is bromo, iodo, carboxyl, or hydroxyl. In another embodiment, the metal coordinating moiety is connected to the bio-directing carrier, either directly or indirectly, via a D group.

Typically, for metal coordinating moieties of Formula (3), X₁, X₂, X₃, X₄ and X₅ are independently methylene optionally substituted by C₁₋₆ alkyl, halo, or hydroxyl.

In another embodiment of metal coordinating moieties of Formula (3), one of Q₁, Q₂, Q₃, Q₄ and Q₅ is substituted by D, while the other four of Q₁, Q₂, Q₃, Q₄ and Q₅ are not substituted by D. Typically, in this embodiment, D is an optionally substituted linker connecting the metal coordinating moiety to one or more bio-directing carriers.

When the metal coordinating moiety corresponds to Formula (3) and m is greater than 0, it is generally preferred that each A be a substituent that positively impacts stability and biodistribution. When present, each A may independently be substituted with one or more aryl, C₁₋₂₀ alkyl, carbaldehyde, keto, carboxyl, cyano, halo, nitro, amido, sulfato, sulfito, phosphato, phosphito, hydroxyl, oxy, ether, C₄-C₂₀ carbohydrate, mercapto, or thio substituents. In addition, when A is aryl or alkyl, each of these, in turn, may be optionally substituted with an aryl or C₁₋₂₀ alkyl moiety optionally substituted with one or more aryl, carbaldehyde, keto, carboxyl, cyano, halo, nitro, amido, sulfato, sulfito, phosphato, phosphito, hydroxyl, oxy, mercapto and thio.

Further, for the metal coordinating moieties of Formula (3), the A substituent, if present, is bonded to any of the backbone carbon atoms. Further, each backbone carbon atom may be substituted by one or two A substituents so that the number of possible A substituents varies with the number of carbon atoms. In one embodiment of metal coordinating moieties of Formula (3) having at least one A substituent, each A is independently aryl or C₁₋₈ alkyl optionally substituted with one or more aryl, keto, carboxyl, cyano, nitro, C₁₋₂₀ alkyl, amido, sulfato, sulfito, phosphato, phosphito, oxy and thio. For example, each A may be aryl or C₁₋₆ alkyl optionally substituted with one or more aryl, keto, amido and oxy. By way of further example, each A may be methyl.

In general, as the value of n increases, the length of the chain of atoms increases. In this manner, the length of the backbone may be controlled to match the size and coordination capacity of the metal to be coordinated.

For any of the above embodiments, the metal coordinating moiety may be complexed with a metal, M, thereby forming a metal complex. For example, in one embodiment where the metal coordinating moiety is a heterocyclic ring and complexed with a metal, M, the complex has the following Formula (4): 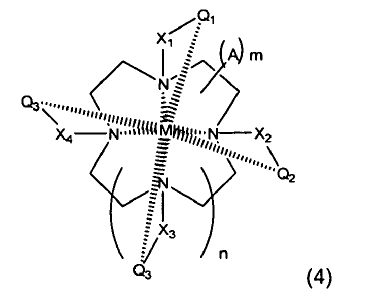 wherein

n is 0, 1 or 2;

m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;

X₁, X₂, X₃ and X₄ are independently optionally substituted methylene where the substituents are selected from the group consisting of aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and

Q₁, Q₂, Q₃ and Q₄ are independently selected from the group consisting of 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D;

each D is independently selected from the group consisting of a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and

M is selected from the group consisting of Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy,Dy-165, Dy-166, Ho, Ho-166, Eu, Eu-169, Sm, Sm-153, Pd, Pd-103, Pm, Pm-149, Tm, Tm-170, Bi, Bi-212, As and As-211.

While not depicted in Formula (4), the hydroxyl or thiol groups of the ((di)thio)dihydroxyaromatic moiety may independently participate in the coordination of the metal. Accordingly, in some embodiments, neither of the hydroxyl or thiol groups directly participate in the coordination of the metal, while in other embodiments one or both of the hydroxyl or thiol groups participate in the coordination of the metal. Both the nature of the metal selected and the particular metal coordinating moiety selected will determine whether the hydroxyl or thiol groups of the resorcinol deriviate participate in the coordination of the metal. Further, when the ((di)thio)dihydroxyaromatic moiety is dihydroxy in nature, both of the oxygen atoms are involved in the bonding of the metal at one time or another due to the quilibrium present. Both hydroxyl oxygens, however, are not bond to the same metal at the same time.

Alternatively, in one embodiment where the metal coordinating moiety comprises a chain of atoms and is complexed with a metal, M, the complex has the following Formula (5): 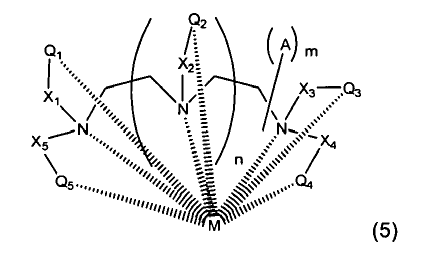 wherein

n is 0, 1 or 2;

m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;

X₁, X₂, X₃, X₄ and X₅ are independently optionally substituted methylene where the substituents are selected from the group consisting of aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and

Q₁, Q₂, Q₃, Q₄, and Q₅ are independently selected from the group consisting of 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D;

each D is independently selected from the group consisting of a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and

M is selected from the group consisting of Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.

While not depicted in Formula (5), the hydroxyl or thiol groups of the ((di)thio)dihydroxyaromatic moiety may independently participate in the coordination of the metal. Accordingly, in some embodiments, neither of the hydroxyl or thiol groups directly participate in the coordination of the metal, while in other embodiments one or both of the hydroxyl or thiol groups participate in the coordination of the metal. Both the nature of the metal selected and the particular metal coordinating moiety selected will determine whether the hydroxyl or thiol groups of the resorcinol deriviate participate in the coordination of the metal. Further, when the ((di)thio)dihydroxyaromatic moiety is dihydroxy in nature, both of the oxygen atoms are involved in the bonding of the metal at one time or another due to the quilibrium present. Both hydroxyl oxygens, however, are not bond to the same metal at the same time.

Whether the preferred complex corresponds to Formula (4) or Formula (5) typically depends on the particular metal selected for coordination. For example, for yttrium and lanthanides, the complex corresponding to Formula (4) is preferred. Formula (4) is also preferred for iron, copper, and manganese while Formula (5) is the preferred complex for the remaining transition metals. The preferred complex for any particular metal is related to the potential for transmetallation with endogenous ion. Thus, Formula (4) provides greater stability with high exchange metals, including, but not limited to, yttrium, lanthanides, and gallium. Transmetallation with endogenous ions does not present as great a concern for regular transition metals.

Macrocyclic metal coordinating moieties with three-dimensional cavities often form metal complexes with high stability. These complexes often exhibit selectivity for certain metal ions based on metal size and coordination chemistry, and capability to adopt a preorganized conformation in the uncomplexed form, which facilitates metal complexation. The selection of appropriate macrocyclic metal coordinating moieties and metals is known by those skilled in the art.

In addition, the preferred value of n, and hence the size or length of the metal coordinating moiety, depends upon the particular metal to be coordinated. For yttrium and lanthanides, for example, n is preferably 1. For transition metals, n is typically 0 or 1. For manganese and technetium, n is 0, 1, or 2 depending on the value of X₁-X₄.

### General Synthesis

The conjugates of the present invention may be synthesized in a variety of ways. For instance, a protected dihydroxybenzyl halide may be used to alkylate a desired polyamine, or a suitable aldehyde derivative may be reductively aminated by a polyamine: 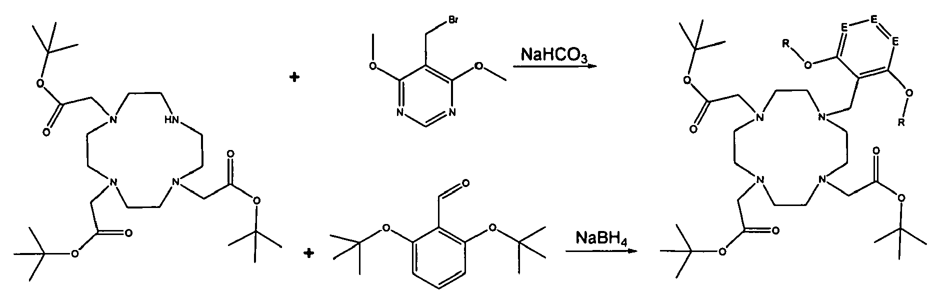 wherein E is as previously defined and R is a hydroxy protecting group (e.g., methyl or t-butyl). The product can then be deprotected as needed. The deprotected ((di)thio)dihydroxyaromatic moiety is then free to coordinate the desired metal ion.

### Metallopharmaceutical Compositions

Metallopharmaceutical compositions of the present invention comprise a conjugate, complexed to a metal, dispersed in a pharmaceutically acceptable carrier. The pharmaceutically acceptable carrier, also known in the art as an excipient, vehicle, auxiliary, adjuvant, or diluent, is typically a substance which is pharmaceutically inert, confers a suitable consistency or form to the composition, and does not diminish the therapeutic or diagnostic efficacy of the conjugate. The carrier is generally considered to be "pharmaceutically or pharmacologically acceptable" if it does not produce an unacceptably adverse, allergic or other untoward reaction when administered to a mammal, especially a human.

The selection of a pharmaceutically acceptable carrier will also, in part, be a function of the route of administration. In general, the metallopharmaceutical compositions of the invention can be formulated for any route of administration so long as the target tissue is available via that route. For example, suitable routes of administration include, but are not limited to, oral, parenteral (e.g., intravenous, intraarterial, subcutaneous, rectal, subcutaneous, intramuscular, intraorbital, intracapsular, intraspinal, intraperitoneal, or intrasternal), topical (nasal, transdermal, intraocular), intravesical, intrathecal, enteral, pulmonary, intralymphatic, intracavital, vaginal, transurethral, intradermal, aural, intramammary, buccal, orthotopic, intratracheal, intralesional, percutaneous, endoscopical, transmucosal, sublingual and intestinal administration.

Pharmaceutically acceptable carriers for use in the compositions of the present invention are well known to those of ordinary skill in the art and are selected based upon a number of factors: the particular conjugate used, and its concentration, stability and intended bioavailability; the disease, disorder or condition being treated or diagnosed with the composition; the subject, its age, size and general condition; and the route of administration. Suitable nonaqueous, pharmaceutically-acceptable polar solvents include, but are not limited to, alcohols (e.g., α-glycerol formal, β-glycerol formal, 1,3-butyleneglycol, aliphatic or aromatic alcohols having 2-30 carbon atoms such as methanol, ethanol, propanol, isopropanol, butanol, t-butanol, hexanol, octanol, amylene hydrate, benzyl alcohol, glycerin (glycerol), glycol, hexylene glycol, tetrahydrofurfuryl alcohol, lauryl alcohol, cetyl alcohol, or stearyl alcohol, fatty acid esters of fatty alcohols such as polyalkylene glycols (e.g., polypropylene glycol, polyethylene glycol), sorbitan, sucrose and cholesterol); amides (e.g., dimethylacetamide (DMA), benzyl benzoate DMA, dimethylformamide, N-(β-hydroxyethyl)-lactamide, N, N-dimethylacetamide_amides, 2-pyrrolidinone, 1-methyl-2-pyrrolidinone, or polyvinylpyrrolidone); esters (e.g., 1-methyl-2-pyrrolidinone, 2-pyrrolidinone, acetate esters such as monoacetin, diacetin, and triacetin, aliphatic or aromatic esters such as ethyl caprylate or octanoate, alkyl oleate, benzyl benzoate, benzyl acetate, dimethylsulfoxide (DMSO), esters of glycerin such as mono, di, or tri-glyceryl citrates or tartrates, ethyl benzoate, ethyl acetate, ethyl carbonate, ethyl lactate, ethyl oleate, fatty acid esters of sorbitan, fatty acid derived PEG esters, glyceryl monostearate, glyceride esters such as mono, di, or tri-glycerides, fatty acid esters such as isopropyl myristrate, fatty acid derived PEG esters such as PEG-hydroxyoleate and PEG-hydroxystearate, N-methyl pyrrolidinone, pluronic 60, polyoxyethylene sorbitol oleic polyesters such as poly(ethoxylated)₃₀₋₆₀ sorbitol poly(oleate)₂₋₄, poly(oxyethylene)₁₅₋₂₀ monooleate, poly(oxyethylene)₁₅₋₂₀ mono 12-hydroxystearate, and poly(oxyethylene)₁₅₋₂₀ mono ricinoleate, polyoxyethylene sorbitan esters such as polyoxyethylene-sorbitan monooleate, polyoxyethylene-sorbitan monopalmitate, polyoxyethylene-sorbitan monolaurate, polyoxyethylene-sorbitan monostearate, and Polysorbate® 20, 40, 60 or 80 from ICI Americas, Wilmington, DE, polyvinylpyrrolidone, alkyleneoxy modified fatty acid esters such as polyoxyl 40 hydrogenated castor oil and polyoxyethylated castor oils (e.g., Cremophor® EL solution or Cremophor® RH 40 solution), saccharide fatty acid esters (i.e., the condensation product of a monosaccharide (e.g., pentoses such as ribose, ribulose, arabinose, xylose, lyxose and xylulose, hexoses such as glucose, fructose, galactose, mannose and sorbose, trioses, tetroses, heptoses, and octoses), disaccharide (e.g., sucrose, maltose, lactose and trehalose) or oligosaccharide or mixture thereof with a C₄-C₂₂ fatty acid(s)(e.g., saturated fatty acids such as caprylic acid, capric acid, lauric acid, myristic acid, palmitic acid and stearic acid, and unsaturated fatty acids such as palmitoleic acid, oleic acid, elaidic acid, erucic acid and linoleic acid)), or steroidal esters); alkyl, aryl, or cyclic ethers having 2-30 carbon atoms (e.g., diethyl ether, tetrahydrofuran, dimethyl isosorbide, diethylene glycol monoethyl ether); glycofurol (tetrahydrofurfuryl alcohol polyethylene glycol ether); ketones having 3-30 carbon atoms (e.g., acetone, methyl ethyl ketone, methyl isobutyl ketone); aliphatic, cycloaliphatic or aromatic hydrocarbons having 4-30 carbon atoms (e.g., benzene, cyclohexane, dichloromethane, dioxolanes, hexane, n-decane, n-dodecane, n-hexane, sulfolane, tetramethylenesulfon, tetramethylenesulfoxide, toluene, dimethylsulfoxide (DMSO), or tetramethylenesulfoxide); oils of mineral, vegetable, animal, essential or synthetic origin (e.g., mineral oils such as aliphatic or wax-based hydrocarbons, aromatic hydrocarbons, mixed aliphatic and aromatic based hydrocarbons, and refined paraffin oil, vegetable oils such as linseed, tung, safflower, soybean, castor, cottonseed, groundnut, rapeseed, coconut, palm, olive, corn, corn germ, sesame, persic and peanut oil and glycerides such as mono-, di- or triglycerides, animal oils such as fish, marine, sperm, cod-liver, haliver, squalene, squalane, and shark liver oil, oleic oils, and polyoxyethylated castor oil); alkyl or aryl halides having 1-30 carbon atoms and optionally more than one halogen substituent; methylene chloride; monoethanolamine; petroleum benzin; trolamine; omega-3 polyunsaturated fatty acids (e.g., alpha-linolenic acid, eicosapentaenoic acid, docosapentaenoic acid, or docosahexaenoic acid); polyglycol ester of 12-hydroxystearic acid and polyethylene glycol (Solutol® HS-15, from BASF, Ludwigshafen, Germany); polyoxyethylene glycerol; sodium laurate; sodium oleate; or sorbitan monooleate.

Other pharmaceutically acceptable solvents for use in the invention are well known to those of ordinary skill in the art, and are identified in The Chemotherapy Source Book (Williams & Wilkens Publishing), The Handbook of Pharmaceutical Excipients, (American Pharmaceutical Association, Washington, D.C., and The Pharmaceutical Society of Great Britain, London, England, 1968), Modern Pharmaceutics, (G. Banker et al., eds., 3d ed.)(Marcel Dekker, Inc., New York, New York, 1995), The Pharmacological Basis of Therapeutics, (Goodman & Gilman, McGraw Hill Publishing), Pharmaceutical Dosage Forms, (H. Lieberman et al., eds.)(Marcel Dekker, Inc., New York, New York, 1980), Remington's Pharmaceutical Sciences (A. Gennaro, ed., 19th ed.)(Mack Publishing, Easton, PA, 1995), The United States Pharmacopeia 24, The National Formulary 19, (National Publishing, Philadelphia, PA, 2000), A.J. Spiegel et al., and Use of Nonaqueous Solvents in Parenteral Products, JOURNAL OF PHARMACEUTICAL SCIENCES, Vol. 52, No. 10, pp. 917-927 (1963).

### Dosage

Dosage and regimens for the administration of the pharmaceutical compositions of the invention can be readily determined by those with ordinary skill in diagnosing or treating disease. It is understood that the dosage of the conjugates will be dependent upon the age, sex, health, and weight of the recipient, kind of concurrent treatment, if any, frequency of treatment, and the nature of the effect desired. For any mode of administration, the actual amount of conjugate delivered, as well as the dosing schedule necessary to achieve the advantageous effects described herein, will also depend, in part, on such factors as the bioavailability of the conjugate, the disorder being treated or diagnosed, the desired therapeutic or diagnostic dose, and other factors that will be apparent to those of skill in the art. The dose administered to an animal, particularly a human, in the context of the present invention should be sufficient to affect the desired therapeutic or diagnostic response in the animal over a reasonable period of time.

Radiolabeled scintigraphic imaging agents provided by the present invention are provided having a suitable amount of radioactivity. In forming diagnostic radioactive complexes, it is generally preferred to form radioactive complexes in solutions containing radioactivity at concentrations of from about 0.01 millicurie (mCi) to 100 mCi per mL. Generally, the unit dose to be administered has a radioactivity of about 0.01 mCi to about 100 mCi, preferably about 1 mCi to about 30 mCi. The solution to be injected at unit dosage is from about 0.01 mL to about 10 mL. The amount of radiolabeled conjugate appropriate for administration is dependent upon the distribution profile of the chosen conjugate in the sense that a rapidly cleared conjugate may need to be administered in higher doses than one that clears less rapidly. *In vivo* distribution and localization can be tracked by standard scintigraphic techniques at an appropriate time subsequent to administration; typically between thirty minutes and 180 minutes depending upon the rate of accumulation at the target site with respect to the rate of clearance at the non-target tissue.

Typically, an In-111 diagnostic dose is 3-6 mCi while a typical Tc-99m does is 10-30 mCi. Generally, radiotherapeutic doses of radiopharmaceuticals vary to a greater extent, depending on the tumor and number of injections of cycles. For example, cumulative doses of Y-90 range from about 100-600 mCi (20 -150 mCi/dose), while cumulative doses of Lu-177 range from about 200-800 mCi (50-200 mCi/dose).

### Kits

For convenience, metallopharmaceutical compositions of the present invention may be provided to the user in the form of a kit containing some or all of the necessary components. The use of a kit is particularly convenient since some of the components, *e*.*g*., a radioisotope, have a limited shelf life, particularly when combined. Thus, the kit may include one or more of the following components (i) a conjugate, (ii) a metal coordinated to or for coordination by the conjugate, (iii) a carrier solution, and (iv) instructions for their combination and use. Depending on the metal, a reducing agent may be desired to prepare the metal for reaction with the conjugate. Exemplary reducing agents include Ce (III), Fe (II), Cu (I), Ti (III), Sb (III), and Sn (II). Of these, Sn (II) is particularly preferred. Often the components of the kit are in unit dosage form (e.g., each component in a separate vial).

For reasons of stability, it may be preferred that the conjugate be provided in a dry, lyophilized state. The user may then reconstitute the conjugate by adding the carrier or other solution.

Because of the short half-life of suitable radionuclides, it will frequently be most convenient to provide the kit to the user without a radionuclide. The radionuclide is then ordered separately when needed for a procedure. Alternatively, if the radionuclide is included in the kit, the kit will most likely be shipped to the user just before it is needed.

In addition to the metal coordinating moiety, biomolecule, active urea, metal and deprotecting acid, the kit of the present invention typically includes a buffer. Exemplary buffers include citrate, phosphate and borate.

The kit optionally contains other components frequently intended to improve the ease of synthesis of the radiopharmaceutical by the practicing end user, the ease of manufacturing the kit, the shelf-life of the kit, or the stability and shelf-life of the radiopharmaceutical. Such components of the present invention include lyophilization aids, e.g., mannitol, lactose, sorbitol, dextran, Ficoll, and polyvinylpyyrolidine (PVP); stabilization aids, e.g., ascorbic acid, cysteine, monothioglycerol, sodium bisulfite, sodium metabisulfite, gentisic acid, and inositol; and bacteriostats, e.g., benzyl alcohol, benzalkonium chloride, chlorbutanol, and methyl, propyl, or butyl paraben.

Typically, when the conjugate is formulated as a kit, the kit comprises multiple vials consisting of a protected metal coordinating moiety having an active urea group, a deprotecting acid, a buffer, and a solution of a radioactive metal such as, but not limited to, In-111, Y-90 or Lu-177. In practice, the user will take the vial containing the metal coordinating moiety and add a solution of a bio-directing carrier of interest bearing a reactive amino (NH₂) group. Once conjugation is complete, the deprotecting acid is added to affect deprotection, followed by addition of the radioactive metal. The mixture is then buffered to complete complexation of the radioactive metal by the metal chelator.

### Definitions

The compounds described herein may have asymmetric centers. Compounds of the present invention containing an asymmetrically substituted atom may be isolated in optically active or racemic form. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated. All processes used to prepare compounds of the present invention and intermediates made therein are considered to be part of the present invention.

The present invention includes all isotopes of atoms occurring in the present compounds. Isotopes include those atoms having the same atomic number but different mass numbers.

Unless otherwise indicated, the alkyl groups described herein are preferably lower alkyl containing from one to eight carbon atoms in the principal chain and up to 20 carbon atoms. They may be straight or branched chain or cyclic and include methyl, ethyl, propyl, isopropyl, butyl, hexyl and the like.

The term "amido" as used herein includes substituted amido moieties where the substituents include, but are not limited to, one or more of aryl and C₁₋₂₀ alkyl, each of which may be optionally substituted by one or more aryl, carbaldehyde, keto, carboxyl, cyano, halo, nitro, C₁₋₂₀ alkyl, sulfato, sulfito, phosphato, phosphito, hydroxyl, oxy, mercapto, and thio substituents.

The term "amino" as used herein includes substituted amino moieties where the substituents include, but are not limited to, one or more of aryl and C₁₋₂₀ alkyl, each of which may be optionally substituted by one or more aryl, carbaldehyde, keto, carboxyl, cyano, halo, nitro, C₁₋₂₀ alkyl, sulfato, sulfito, phosphato, phosphito, hydroxyl, oxy, mercapto, and thio substituents.

The term "aromatic" as used herein alone or as part of another group denote optionally substituted homo- or heterocyclic aromatic groups. These aromatic groups are preferably monocyclic, bicyclic, or tricyclic groups containing from 6 to 14 atoms in the ring portion. The term "aromatic" encompasses the "aryl" and "heteroaryl" groups defined below.

The terms "aryl" or "ar" as used herein alone or as part of another group denote optionally substituted homocyclic aromatic groups, preferably monocyclic or bicyclic groups containing from 6 to 12 carbons in the ring portion, such as phenyl, biphenyl, naphthyl, substituted phenyl, substituted biphenyl or substituted naphthyl. Phenyl and substituted phenyl are the more preferred aryl.

The term "complex" refers to a metal coordinating moiety of the invention, e.g. Formula (1), complexed or coordinated with a metal. The metal is typically a radioactive isotope or paramagnetic metal ion.

The term "conjugate" refers to a metal coordinating moiety of the invention, e.g. Formula (1), bonded to a bio-directing carrier (biomolecule) whether or not the metal coordinating moiety is complexed with a metal. For the present invention, the metal coordinating moiety is bonded to the bio-directing carrier directly or indirectly via a linker.

The terms "halogen" or "halo" as used herein alone or as part of another group refer to chlorine, bromine, fluorine, and iodine.

The term "heteroaryl" as used herein alone or as part of another group denote optionally substituted aromatic groups having at least one heteroatom in at least one ring, and preferably 5 or 6 atoms in each ring. The heteroaryl group preferably has 1 or 2 oxygen atoms and/or 1 to 4 nitrogen atoms and/or 1 or 2 sulfur atoms in the ring, and is bonded to the remainder of the molecule through a carbon. Exemplary heteroaryls include furyl, thienyl, pyridyl, oxazolyl, isoxazolyl, oxadiazolyl, pyrrolyl, pyrazolyl, triazolyl, tetrazolyl, imidazolyl, pyrazinyl, pyrimidyl, pyridazinyl, thiazolyl, thiadiazolyl, biphenyl, naphthyl, indolyl, isoindolyl, indazolyl, quinolinyl, isoquinolinyl, benzimidazolyl, benzotriazolyl, imidazopyridinyl, benzothiazolyl, benzothiadiazolyl, benzoxazolyl, benzoxadiazolyl, benzothienyl, benzofuryl and the like.

The term "heteroatom" shall mean atoms other than carbon and hydrogen.

The terms "heterocyclo" or "heterocyclic" as used herein alone or as part of another group denote optionally substituted, fully saturated or unsaturated, monocyclic or bicyclic, aromatic or nonaromatic groups having at least one heteroatom in at least one ring. The heterocyclo group preferably has 1 to 5 nitrogen atoms in the ring, and may be bonded to the remainder of the molecule through a carbon atom. Exemplary heterocyclics include macrocyclics, cyclen, DOTA, DOTMA, DOTP, and TETA.

The "heterosubstituted alkyl" moieties described herein are alkyl groups in which a carbon atom is covalently bonded to at least one heteroatom and optionally with hydrogen, the heteroatom being, for example, a nitrogen atom.

The term "metallopharmaceutical" as used herein refers to a pharmaceutically acceptable compound comprising a metal, wherein the compound is useful for imaging or treatment.

The term "hydroxy protecting group" as used herein denotes a group capable of protecting a free hydroxyl group ("protected hydroxyl") which, subsequent to the reaction for which the protection is employed, may be removed without disturbing the remainder the molecule Any conventional means of protecting the hydroxyl groups is permissible. A variety of protecting groups for the hydroxyl groups and the synthesis thereof may be found in Protective Groups in Organic Synthesis, 3rd Edition by T.W. Greene and P.G.M. Wuts, John Wiley and Sons, 1999.

### Examples

The following examples are prophetic.

### Example 1: Vitamin B12 Conjugate

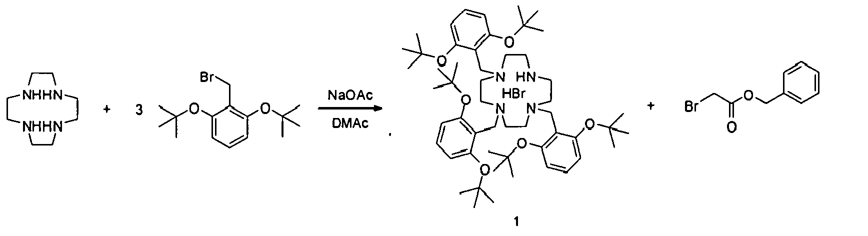

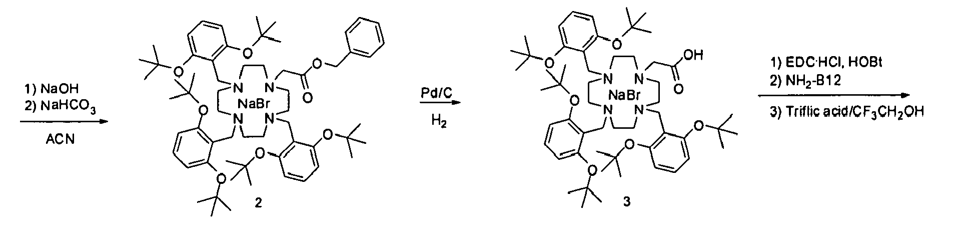

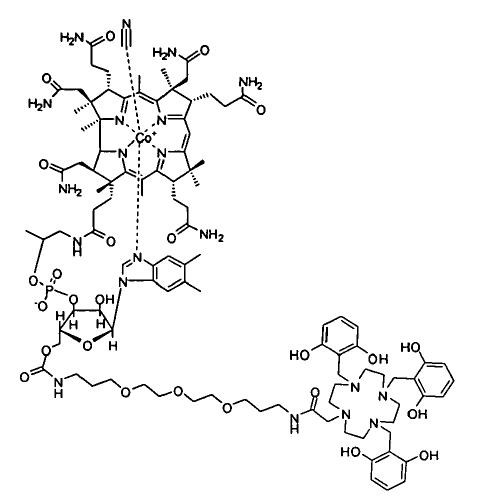

In this example, cyclen is allowed to react with 3 equivalents of 2-(bromomethyl)-1,3-di-tert-butoxybenzene and sodium acetate in dimethylacetamide. The resulting trisubstituted macrocycle, **1**, may be isolated by filtration, dissolved in chloroform and washed with saturated aqueous sodium bromide. The organic extract is treated with hexane to affect crystallization of the product. The secondary amine, **1**, is free-based with aqueous sodium hydroxide and dissolved in ether. The organic extract is collected, dried with magnesium sulfate and concentrated to an oil. The oil is dissolved in acetonitrile, treated with sodium bicarbonate and benzyl bromoacetate. The resulting product, **2**, may be isolated by crystallization from acetonitrile. The benzyl ester is then selectively removed by treatment with palladium on carbon and hydrogen in methanol. The carboxylic acid, **3**, is conjugated to B12-(2-yl)methyl 3-(2-(2-(3-aminopropoxy)ethoxy)eth-oxy)propylcarbamate, or B12-NH₂, by treatment with 1-[3-(dimethylamino)propyl]-3-ethylcarbodiimide hydrochloride and 1-hydroxybenzotriazole hydrate in dimethylformamide. The protected intermediate is then treated with a mixture of triflic acid and trifluorethanol to affect de-t-butylation. The unmasked chelate may be purified by reverse phase chromatography.

### Example 2: A Glucose Derivative Conjugate

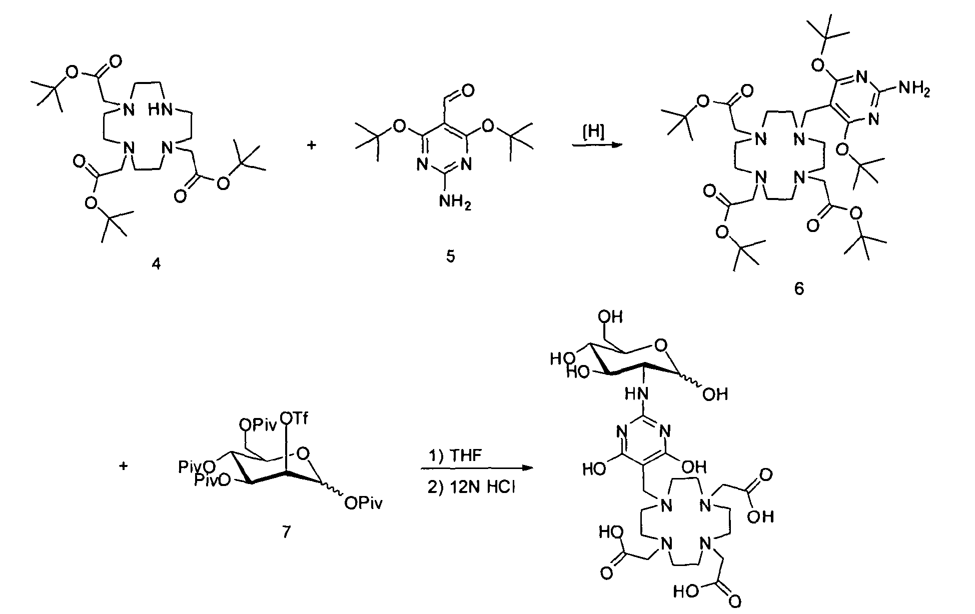

In this example, 2-Amino-4,6-di-tert-butoxypyrimidine-5-carbaldehyde, **5**, is reductively aminated with tert-butyl 2,2',2"-(1,4,7,10-tetraazacyclododecane-1,4,7-triyl)triacetate, or DO3A-tris(t-butyl ester), **4**, and sodium cyanoborohydride. The protected intermediate, **6**, is further derivatized by allowing it to react with (3R,4S,5R)-6-(pivaloyloxymethyl)-3-(trifluoromethylsulfonyloxy)tetrahydro-2H-pyran-2,4,5-triyl tris(2,2-dimethylpropanoate), **7**. The masked intermediate can be deprotected by treatment with strong acid, and isolated by reverse phase chromatography.

### Example 3: A Bombesin Conjugate

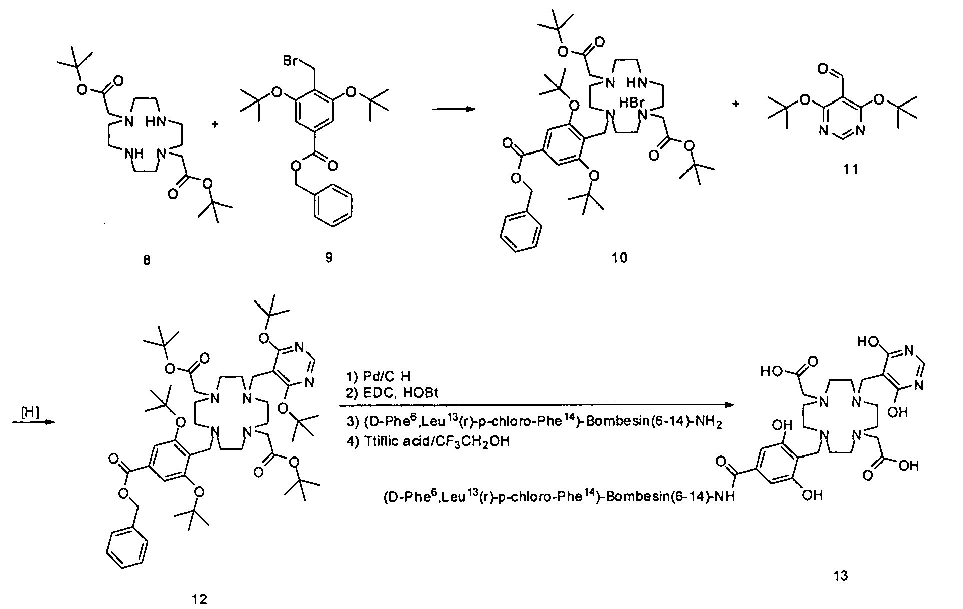

In this example, tert-Butyl 2,2'-(1,4,7,10-tetraazacyclododecane-1,7-diyl)diacetate, **8**, is monoalkylated by treatment with benzyl 4-(bromomethyl)-3,5-di-tert-butoxybenzoate, **9**, and sodium acetate in DMAc. The product, tert-butyl 2,2'-(4-(4-(benzyloxycarbonyl)-2,6-di-tert-butoxybenzyl)-1,4,7,10-tetraazacyclododecane-1,7-diyl)diacetate, **10**, after free-basing, is alkylated with 4,6-di-tert-butoxypyrimidine-5-carbaldehyde by treatment with sodium cyanoborohydride. The masked intermediate, tert-butyl 2,2'-(4-(4-(benzyloxycarbonyl)-2,6-di-tert-butoxybenzyl)-10-((4,6-di-tert-butoxypyrimidin-5-yl)methyl)-1,4,7,10-tetraazacyclododecane-1,7-diyl)diacetate, **12**, after partial deprotection by hydrogenolysis, acylates the N-terminus of (D-Phe⁶,Leu¹³(r)-p-chloro-Phe¹⁴)-Bombesin(6-14)-NH₂. Complete deprotection is accomplished by means of triflic acid in trifluoroethanol.

The invention comprises the following features:
1. A conjugate comprising a bio-directing carrier, a metal coordinating moiety, and a linker chemically linking the metal coordinating moiety to the carrier, the metal coordinating moiety comprising at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl moiety, or a combination thereof.
2. The conjugate of feature 1 wherein the bio-directing carrier is selected from imidazole, triazole, antibodies, proteins, peptides, carbohydrates, vitamins, hormones, drugs, and small organic molecules.
3. The conjugate of feature 1 wherein the conjugate comprises more than one bio-directing carrier.
4. The conjugate of features 1-3 wherein the metal coordinating moiety is a polycarboxylic acid.
5. The conjugate of feature 4 wherein the metal coordinating moiety is selected from EDTA, DTPA, DCTA, DOTA, TETA, or analogs or homologs thereof.
6. The conjugate of features 1-3 wherein the metal coordinating moiety is a triaza- or tetraza-macrocycle.
7. The conjugate of any of features 1-6 wherein the metal coordinating moiety is complexed with a metal, the metal consisting of a radioisotope or a paramagnetic metal.
8. The conjugate of feature 7 wherein the metal is selected from Lu, Lu-177, Y, Y-90, In, In-111 , Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.
9. The conjugate of feature 1 wherein the metal coordinating moiety comprises a substituted heterocyclic ring.
10. The conjugate of feature 9 wherein said heterocyclic ring comprises 9 to 15 ring atoms, at least 3 of said ring atoms being nitrogen.
11. The conjugate of feature 9 or 10 wherein said heterocyclic ring comprises 3-5 ring nitrogen atoms.
12. The conjugate of any of features 9-11 wherein said heterocyclic ring is optionally substituted at one or more ring carbon atoms.
13. The conjugate of feature 12 wherein said heterocyclic ring is substituted at one or more ring nitrogen atoms.
14. The conjugate of feature 1 wherein the metal coordinating moiety comprises a substituted heterocyclic ring having the following structure: 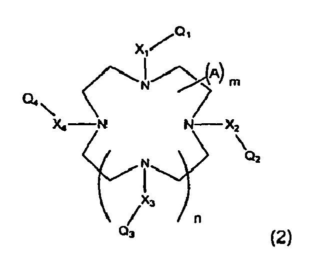 wherein
   n is 0, 1 or 2;
   m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁, X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and
   Q₁, Q₂, Q₃ and Q₄ are independently selected from 1-hydroxyphenyl, 1- thiolphenyl, 1,3-dihydroxyphenyl, 3,5-dihyroxypyridyl, 2,4-dihydroxypyridyl, 4,6-dihydroxypyrimidyl, 1,3- dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1 ,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6- dithiolpyrimidyl, 1-hydroxy-3-thiolphenyl, 3-hydroxy-5-thiolpyridyl, 2-hydroxy-4-thiolpyridyl, 4-hydroxy- 6-thiolpyrimidyl, 1-thiol-3-hydroxyphenyl, 3-thiol-5-hydroxypyridyl, 2-thiol-4-hydroxypyridyl, or 4-thiol-6- hydroxypyrimidyl and (b) Q₁, Q₂, Q₃ and Q₄ are optionally substituted at each substitutable carbon atom by D; and
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.
15. The conjugate of feature 1 wherein the metal coordinating moiety comprises a substituted chain of carbon and nitrogen atoms.
16. The conjugate of feature 15 wherein said substituted chain comprises 4 to 10 atoms, at least 2 of said atoms being nitrogen.
17. The conjugate of features 15 or 16 wherein said substituted chain comprises 2-4 nitrogen atoms.
18. The conjugate of any of features 15-17 wherein said substituted chain is optionally substituted at one or more carbon atoms.
19. The conjugate of feature 18 wherein said substituted chain is substituted at one or more nitrogen atoms.
20. The conjugate of feature 1 wherein the metal coordinating moiety comprises a substituted chain of carbon and nitrogen atoms having the following structure: 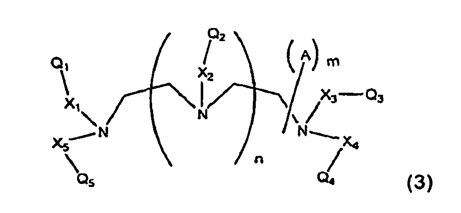 wherein
   n is O, 1 or 2;
   m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁, X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and
   Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6- dihydroxypyrimidyl, 1,3-dithiolphenyl, 3,5-dithiolpyridyl, 2,4-dithiolpyridyl, 4,6-dithiolpyrimidyl, 1-hydroxy- 3-thiolphenyl, 3-hydroxy-5-thiolpyridyl, 2-hydroxy-4-thiolpyridyl, 4-hydroxy-6-thiolpyrimidyl, 1-thiol-3- hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄ and Q₅ is 1,3-dihydroxyphenyl, 3,5-dihyroxypyridyl, 2,4-dihydroxypyridyl, 4,6-dihydroxypyrimidyl, 1,3-dithiolphenyl, 3,5- dithiolpyridyl, 2,4-dithiolpyridyl, 4,6-dithiolpyrimidyl, 1-hydroxy-3-thiolphenyl, 3-hydroxy-5-thiolpyridyl, 2- hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D; and
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.
21. The conjugate of feature 1 wherein the linker is selected from C₁₋₁₀ alkylene, oxygen, sulfur, keto, amino, amido, urea, thiourea, and ester, the alkylene, amino, amido, urea, and thiourea groups being optionally substituted with aryl, C₁₋₇ alkyl, C₁₋₇ hydroxyalkyl or C₁₋₇ alkoxyalkyl.
22. The conjugate of feature 21 wherein the linker is selected from the group consisting of C₁₋₁₀ alkylene, oxygen, sulfur, keto, amino, amido, thiourea, and ester.
23. The conjugate of feature 1 wherein the metal coordinating moiety is complexed with a metal, M, forming a metal complex having the formula 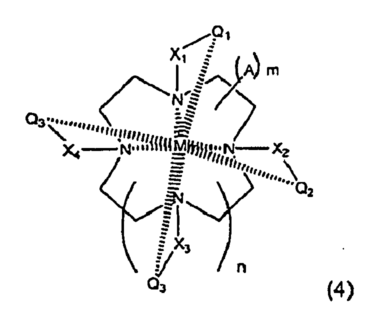 wherein
   n is 0, 1 or 2;
   m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁ X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
   Q₁, Q₂, Q₃ and Q₄ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1 ,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D;
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and
   M is selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy,Dy-165, Dy-166, Ho, Ho-166, Eu, Eu-169, Sm, Sm-153, Pd, Pd-103, Pm, Pm- 149, Tm, Tm-170, Bi, Bi-212, As and As-211.
24. The conjugate of feature 1 wherein the metal coordinating moiety is complexed with a metal, M, forming a metal complex having the formula 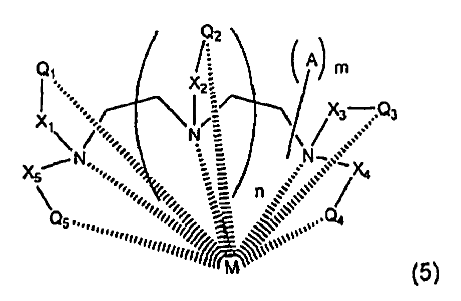 wherein
   n is 0, 1 or 2;
   m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁ X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
   Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy- 3-thiolphenyl, 3-hydroxy-5-thiolpyridyl, 2-hydroxy-4-thiolpyridyl, 4-hydroxy-6-thiolpyrimidyl, 1-thiol-3- hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄, and Q₅ is 1,3-dihydroxyphenyl, 3,5-dihyroxypyridyl, 2,4-dihydroxypyridyl, 4,6-dihydroxypyrimidyl, 1,3-dithiolphenyl, 3,5- dithiolpyridyl, 2,4-dithiolpyridyl, 4,6-dithiolpyrimidyl, 1-hydroxy-3-thiolphenyl, 3-hydroxy-5-thiolpyridyl, 2- hydroxy-4-thiolpyridyl, 4-hydroxy-6-thiolpyrimidyl, 1-thiol-3-hydroxyphenyl, 3-thiol-5-hydroxypyridyl, 2- thiol-4-hydroxypyridyl, or 4-thiol-6-hydroxypyrimidyl and (b) Q₁, Q₂, Q₃ and Q₄ are optionally substituted at each substitutable carbon atom by D;
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and
   M is selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.
25. A pharmaceutical composition comprising the conjugate of any of features 1-24 and a pharmaceutically acceptable carrier.
26. A method for the diagnosis of cancer in a mammal, the method comprising administering to said mammal an effective amount of the conjugate of any of features 1-24 for the diagnosis of cancer and a pharmaceutically acceptable carrier.
27. A method for treating cancer in a mammal, the method comprising administering to said mammal an effective amount of the conjugate of any of features 1-24 and a pharmaceutically acceptable carrier.
28. A kit comprising a metal coordinating moiety comprising at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy- 5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, or 4-thiol-6-hydroxypyrimidyl moiety, or a combination thereof, a reactive electrophile, a deprotecting acid, and a buffer wherein the metal coordinating moiety comprises one of the following structures: 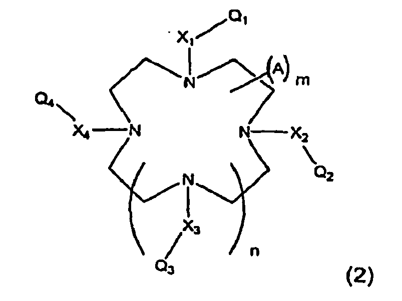 wherein
   n is O, 1 or 2;
   m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁, X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
   Q₁, Q₂, Q₃ and Q₄ are independently selected from 1-hydroxyphenyl, 1- thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy- 5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy- 6-thiolpyrimidyl, 1-thiol-3-hydroxyphenyl, 3-thiol-5-hydroxypyridyl, 2-thiol-4-hydroxypyridyl, or 4-thiol-6-hydroxypyrimidyl and (b) Q₁, Q₂, Q₃ and Q₄ are optionally substituted at each substitutable carbon atom by D; and
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; or 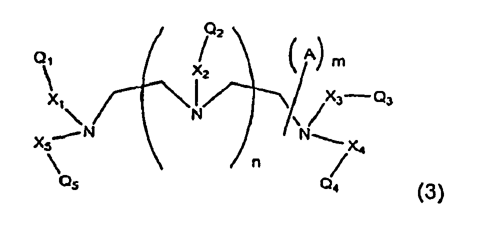
   wherein
   n is 0, 1 or 2;
   m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
   X₁, X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
   Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄, and Q₅ is 1,3-dihydroxyphenyl, 3,5-dihyroxypyridyl, 2,4-dihydroxypyridyl, 4,6-dihydroxypyrimidyl, 1,3-dithiolphenyl, 3,5- dithiolpyridyl, 2,4-dithiolpyridyl, 4,6-dithiolpyrimidyl, 1-hydroxy-3-thiolphenyl, 3-hydroxy-5-thiolpyridyl, 2- hydroxy-4-thiolpyridyl, 4-hydroxy-6-thiolpyrimidyl, 1-thiol-3-hydroxyphenyl, 3-thiol-5-hydroxypyridyl, 2- thiol-4-hydroxypyridyl, or 4-thiol-6-hydroxypyrimidyl and (b) Q₁, Q₂, Q₃ and Q₄ are optionally substituted at each substitutable carbon atom by D; and
   each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.
29. The kit of feature 28 wherein the buffer is selected from citrate, phosphate and borate.
30. The kit of feature 28 or feature 29 wherein the reactive electrophite is selcted from active urea, active ester, and active alkyhalide.
31. The kit of any of features 28-30 wherein the metal coordinating moiety, the reactive electrophile, the deprotecting acid, and the buffer are in unit dosage form.
32. The kit of any of features 28-31 wherein the kit additionally comprises a solution of a radioactive metal.
33. The kit of feature 32 wherein the radioactive metal is selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.

## Claims

1. A conjugate comprising a bio-directing carrier, a metal coordinating moiety, and a linker chemically linking the metal coordinating moiety to the carrier, the metal coordinating moiety comprising at least one optionally substituted 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl, 2-thiol-4-hydroxypyridyl , 1,3-dithiolphenyl, 1-hydroxy-3-thiolphenyl or 1-thiol-3-hydroxyphenyl moiety, or a combination thereof.

2. The conjugate of claim 1 wherein the bio-directing carrier is selected from imidazole, triazole, antibodies, proteins, peptides, carbohydrates, vitamins, hormones, drugs, and small organic molecules.

3. The conjugate of claim 1 wherein the conjugate comprises more than one bio-directing carrier.

4. The conjugate of claims 1-3 wherein the metal coordinating moiety includes a polycarboxylic acid, wherein the carboxylic acid is preferably selected from the group consisting of EDTA, DTPA, DCTA, DOTA, TETA, or analogs or homologs thereof.

5. The conjugate of claims 1-3 wherein the metal coordinating moiety includes a triaza- or tetraza-macrocycle.

6. The conjugate of any of claims 1-5 wherein the metal coordinating moiety is complexed with a metal, the metal consisting of a radioisotope or a paramagnetic metal, wherein the metal is preferably selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.

7. The conjugate of claim 1 wherein the metal coordinating moiety comprises a substituted heterocyclic ring having the following structure: 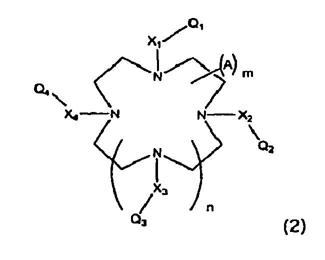 wherein
n is 0, 1 or 2;
m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁, X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and
Q₁, Q₂, Q₃ and Q₄ are independently selected from 1-hydroxyphenyl, 1- thiolphenyl, 1,3-dihydroxyphenyl, 3,5-dihyroxypyridyl, 2,4-dihydroxypyridyl, 4,6-dihydroxypyrimidyl, 1,3- dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl, 2-thiol-4-hydroxypyridyl, 1,3-dithiolphenyl, 1-hydroxy-3-thiolphenyl or 1-thiol-3-hydroxyphenyl and (b) Q₁, Q₂, Q₃ and Q₄ are optionally substituted at each substitutable carbon atom by D; and each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.

8. The conjugate of claim 1 wherein the metal coordinating moiety comprises a substituted chain of carbon and nitrogen atoms having the following structure: 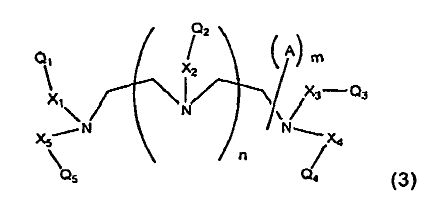 wherein
n is O, 1 or 2;
m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁, X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio; and
Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6- dihydroxypyrimidyl, 1,3-dithiolphenyl, 3,5-dithiolpyridyl, 2,4-dithiolpyridyl. 4,6-dithiolpyrimidyl, 1-hydroxy- 3-thiolphenyl, 3-hydroxy-5-thiolpyridyl, 2-hydroxy-4-thiolpyridyl, 4-hydroxy-6-thiolpyrimidyl, 1-thiol-3- hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄ and Q₅ is 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl, 2-thiol-4-hydroxypyridyl, 1,3-dithiolphenyl, 1-hydroxy-3-thiolphenyl or 1-thiol-3-hydroxyphenyl and (b) Q₁, Q₂, Q₃ and Q₄ are optionally substituted at each substitutable carbon atom by D; and
each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.

9. The conjugate according to any preceding claim wherein the linker is selected from C₁₋₂₀ alkylene, oxygen, sulfur, keto, amino, amido, urea, thiourea, and ester, the alkylene, amino, amido, urea, and thiourea groups being optionally substituted with aryl, C₁₋₇ alkyl, C₁₋₇ hydroxyalkyl or C₁₋₇ alkoxyalkyl.

10. The conjugate of claim 9 wherein the linker is selected from the group consisting of C₁₋₁₀ alkylene, oxygen, sulfur, keto, amino, amido, thiourea, and ester.

11. The conjugate of claim 1 wherein the metal coordinating moiety is complexed with a metal, M, forming a metal complex having the formula 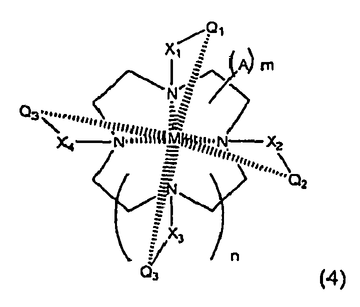 wherein
n is 0, 1 or 2;
m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁ X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
Q₁, Q₂, Q₃ and Q₄ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3- dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl , 2-thiol-4-hydroxypyridyl, 1,3-dithiolphenyl, 1-hydroxy-3-thiolphenyl or 1-thiol-3-hydroxyphenyl and (b) Q₁, Q₂, Q₃ and Q₄ are optionally substituted at each substitutable carbon atom by D;
each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and
M is selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy,Dy-165, Dy-166, Ho, Ho-166, Eu, Eu-169, Sm, Sm-153, Pd, Pd-103, Pm, Pm- 149, Tm, Tm-170, Bi, Bi-212, As and As-211.

12. The conjugate of claim 1 wherein the metal coordinating moiety is complexed with a metal, M, forming a metal complex having the formula 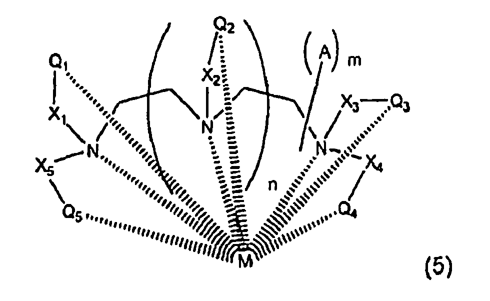 wherein
n is 0, 1 or 2;
m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁ X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy- 3-thiolphenyl, 3-hydroxy-5-thiolpyridyl, 2-hydroxy-4-thiolpyridyl, 4-hydroxy-6-thiolpyrimidyl, 1-thiol-3- hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄, and Q₅ is 4,6-dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl, 2-thiol-4-hydroxypyridyl, 1,3-dithiolphenyl, 1-hydroxy-3-thiolphenyl or 1-thiol-3-hydroxyphenyl and (b) Q₁, Q₂, Q₃ and Q₄ are optionally substituted at each substitutable carbon atom by D;
each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; and
M is selected from Lu, Lu-177, Y, Y-90, In, In-111, Tc, Tc=O, Tc-99m, Tc-99m=O, Re, Re-186, Re-188, Re=O, Re-186=O, Re-188=O, Ga, Ga-67, Ga-68, Cu, Cu-62, Cu-64, Cu-67, Gd, Gd-153, Dy, Dy-165, Dy-166, Ho, Ho-166, Eu, Eu-169, Sm, Sm-153, Pd, Pd-103, Pm, Pm-149, Tm, Tm-170, Bi, Bi-212, As and As-211.

13. A pharmaceutical composition comprising the conjugate of any of claims 1-12 and a pharmaceutically acceptable carrier.

14. A conjugate according to any preceding claim, for use in the diagnosis or treatment of cancer.

15. A kit comprising a metal coordinating moiety comprising at least one optionally substituted 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy- 5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, or 4-thiol-6-hydroxypyrimidyl moiety, or a combination thereof, a reactive electrophile, a deprotecting acid, and a buffer wherein the metal coordinating moiety comprises one of the following structures: 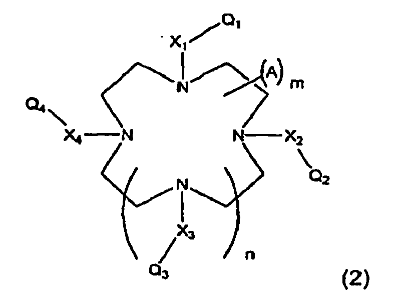 wherein
n is O, 1 or 2;
m is 0-20 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁, X₂, X₃ and X₄ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
Q₁, Q₂, Q₃ and Q₄ are independently selected from 1-hydroxyphenyl, 1- thiolphenyl, 1,3-dihydroxyphenyl, 3,5-dihyroxypyridyl, 2,4-dihydroxypyridyl, 4,6-dihydroxypyrimidyl, 1,3- dithiolphenyl, 3,5-dithiolpyridyl, 2,4-dithiolpyridyl, 4,6-dithiolpyrimidyl, 1-hydroxy-3-thiolphenyl, 3-hydroxy- 5-thiolpyridyl, 2-hydroxy-4-thiolpyridyl, 4-hydroxy-6-thiolpyrimidyl, 1-thiol-3-hydroxyphenyl, 3-thiol-5- hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃ and Q₄ is 4,6- dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl , 2-thiol-4-hydroxypyridyl, 1,3-dithiolphenyl, 1-hydroxy-3-thiolphenyl or 1-thiol-3-hydroxyphenyl and (b) Q₁, Q₂, Q₃ and Q₄ are optionally substituted at each substitutable carbon atom by D; and
each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito; or 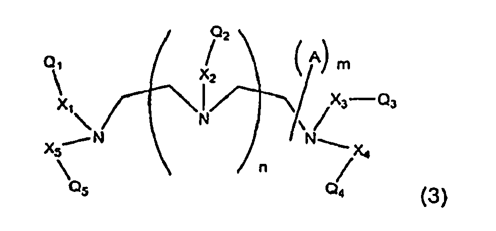
wherein
n is 0, 1 or 2;
m is 0-12 wherein when m is greater than 0, each A is C₁₋₂₀ alkyl or aryl optionally substituted by one or more aryl, C₁₋₂₀ alkyl, carbaldehyde, keto, carboxyl, cyano, halo, nitro, amido, sulfato, sulfito, phosphato, phosphito, hydroxyl, oxy, ether, mercapto or thio;
X₁, X₂, X₃, X₄ and X₅ are independently optionally substituted methylene where the substituents are selected from aryl, C₁₋₂₀ alkyl, carbaldehyde, keto, carboxyl, cyano, halo, nitro, amido, sulfato, sulfito, phosphato, phosphito, hydroxyl, oxy, ether, mercapto and thio;
Q₁, Q₂, Q₃, Q₄, and Q₅ are independently selected from 1-hydroxyphenyl, 1-thiolphenyl, 1,3-dihydroxyphenyl, 3,5-dihyroxypyridyl, 2,4-dihydroxypyridyl, 4,6-dihydroxypyrimidyl, 1,3-dithiolphenyl, 3,5-dithiolpyridyl, 2,4-dithiolpyridyl, 4,6-dithiolpyrimidyl, 1-hydroxy-3-thiolphenyl, 3-hydroxy-5-thiolpyridyl, 2-hydroxy-4-thiolpyridyl, 4-hydroxy-6-thiolpyrimidyl, 1-thiol-3-hydroxyphenyl, 3-thiol-5-hydroxypyridyl, 2-thiol-4-hydroxypyridyl, 4-thiol-6-hydroxypyrimidyl, methylthio, carboxyl, phosphanate, and sulfonate wherein (a) at least one of Q₁, Q₂, Q₃, Q₄, and Q₅ is 4,6- dihydroxypyrimidyl, 4,6-dithiolpyrimidyl, 4-thiol-6-hydroxypyrimidyl, 4-hydroxy-6-thiolpyrimidyl, 3,5-dihyroxypyridyl, 2,4-dihydroxypyridyl, 3,5-dithiolpyridyl, 2,4-dithiolpyridyl, 3-hydroxy-5-thiolpyridyl, 2-hydroxy-4-thiolpyridyl, 3-thiol-5-hydroxypyridyl , 2-thiol-4-hydroxypyridyl, 1,3-dithiolphenyl, 1-hydroxy-3-thiolphenyl or 1-thiol-3-hydroxyphenyl I and (b) Q₁, Q₂, Q₃ and Q₄ are optionally substituted at each substitutable carbon atom by D; and
each D is independently selected from a linker connecting the metal coordinating moiety to a bio-directing carrier, fluoro, chloro, bromo, iodo, carboxyl, cyano, nitro, amido, hydroxyl, amino, sulfato, sulfito, phosphato, phosphito, ether, aryl, and C₁₋₂₀ alkyl optionally substituted with one or more of C₁₋₂₀ alkyl, carboxyl, cyano, nitro, amido, hydroxyl, amino, sulfato, sulfito, phosphato, and phosphito.
